# FASCICULE DE BREVET EUROPEEN

(11) **EP 2 125 992 B1**
(45) Date de publication et mention de la délivrance du brevet: **12.09.2012**
(21) Numéro de dépôt: 08805659.3
(22) Date de dépôt: 21.03.2008
(51) Int. Cl.: C09K 11/02, C09K 11/06, G01N 33/58

(54) **NANOCRISTAUX ORGANIQUES LUMINESCENTS POUR LA REALISATION DE CAPTEURS BIOLOGIQUES**
FLUORESZIERENDE ORGANISCHE NANOKRISTALLE ZUR HERSTELLUNG VON BIOSENSOREN
FLUORESCENT ORGANIC NANOCRYSTALS FOR PRODUCING BIOSENSORS

(30) Priorité: 21.03.2007 FR 0753947
(43) Date de publication de la demande: 02.12.2009
(73) Titulaire: Centre National de la Recherche Scientifique, 75794 Paris Cedex 16 (FR)
(72) Inventeur: IBANEZ, Alain, F-38500 Voiron (FR); MONNIER, Virginie, 69130 ECULLY (FR); SANZ, Nathalie, 29460 IRVILLAC (FR); PANSU, Robert, F-91120 Palaiseau (FR)
(74) Mandataire: Novagraaf Technologies
(86) Numéro de dépôt international: PCT/FR2008/050490
(87) Numéro de publication internationale: WO 2008/145875

(56) Documents cités:
- EP-A- 1 541 656
- US-B1- 6 770 220
- HE H ET AL: "Synthesis and room temperature photoluminescence of AgI nanoparticles embedded in silica sol-gel coating" SOLID STATE IONICS, NORTH HOLLAND PUB. COMPANY. AMSTERDAM, NL, vol. 175, no. 1-4, 30 novembre 2004 (2004-11-30), pages 651-654, XP004667657 ISSN: 0167-2738
- BOTZUNG-APPERT ET AL: "Spatial control of organic nanocrystal nucleation in sol-gel thin films for 3-D optical data storage devices or chemical multi-sensors" JOURNAL OF CRYSTAL GROWTH, ELSEVIER, AMSTERDAM, NL, vol. 283, no. 3-4, 1 octobre 2005 (2005-10-01), pages 444-449, XP005065765 ISSN: 0022-0248

## Description

### Priorité

La présente invention revendique le droit de priorité à la demande de brevet français N° 07/53947 déposée le 21 mars 2007.

### Domaine technique

La présente invention concerne le domaine des matériaux à propriétés optiques, et en particulier des capteurs et des senseurs, notamment biologiques. Tout particulièrement il peut s'agir de puces, notamment de biopuces, en particulier à fluorescence.

Dans la description ci-dessous, les références entre crochets ([ ]) renvoient à la liste des références présentées après les exemples.

### Etat de la technique

Les biopuces sont des outils majeurs dans la recherche clinique, en particulier pour le développement de tests diagnostics et pronostics, ainsi que dans la recherche de nouvelles procédures thérapeutiques.

Les biopuces les plus couramment utilisées sont les biopuces à fluorescence. Cependant, celles-ci peuvent présenter une sensibilité insuffisante pour procéder à l'analyse d'échantillons précieux et chers, fragiles et/ou compliquées à fabriquer. Les biopuces peuvent encore être insuffisamment transparentes, en particulier dans certaines longueurs d'ondes, comme l'Infra-Rouge. Elles peuvent également être insuffisamment stables, et par exemple ne pas présenter une résistance chimique ou temporelle satisfaisante.

Tout particulièrement les procédés permettant de préparer les matériaux permettant de fabriquer des puces, et notamment des biopuces, ou de préparer les puces sont généralement onéreux, difficiles à être mis en place de manière « industrielle » (en particulier pour préparer un grand nombre de puces en même temps et/ou en une seule opération), délicats et/ou complexes à mettre en oeuvre.

Il existe donc un besoin pour des puces qui permettent de résoudre en tout ou partie les problèmes évoqués ci-dessus, notamment en termes de résistance, en particulier thermique, mécanique, chimique et/ou photochimique ; et/ou des biopuces qui permettent de présenter de bonnes propriétés optiques, comme la fluorescence, une bonne stabilité, une grande gamme de transparence, ainsi qu'un procédé de fabrication et de mise en forme aisés, une faible diffusion et une bonne stabilité.

Il existe d'autre part également un besoin pour un (nano)matériau permettant de préparer des puces permettant de résoudre en tout ou partie les problèmes évoqués ci-dessus.

Il existe encore un besoin pour un procédé de préparation de ce (nano)matériau permettant de résoudre en tout ou partie les problèmes évoqués ci-dessus.

Haiping He et al, Solid State Ionics, vol. 175, no. 1-4, pages 651-654, décrit des nanocristaux d'iodure d'argent intégrés dans une matrice sol gel de type silicate et étant en contact direct avec le milieu extérieur.

Botzung-Appert et al, Journal of Crystal Growth, vol. 283, no. 3-4, pages 444-449, décrit une matrice sol gel de variété silicate se présentant sous forme de film d'une épaisseur de 500 nm dans laquelle est intégré au moins un nanocristal luminescent.

### Description de l'invention

Ainsi, selon un premier aspect, l'invention a pour objet un (nano)matériau comprenant au moins une couche inorganique et/ou organo-minérale, dans laquelle est intégré au moins un type de nano-cristal fluorescent dont au moins une partie est en contact direct avec le milieu extérieur, dans laquel ledit au moins un type de nano-cristal émerge de 2 à 50 % de son diamètre par rapport à la cache inorganique et/ou organes - minérale.

Dans un mode de réalisation, le (nano)matériau comprend au moins une couche inorganique et/ou organo-minérale, dans laquelle est intégré au moins un type de nano-cristal fluorescent organique ou organo-métallique émergeant de la surface de ladite couche de sorte qu'au moins une partie du nano-cristal est en contact direct avec le milieu extérieur.

Par « (nano)matériau », on entend au sens de la présente invention un matériau ou un nanomatériau tel que défini ci-dessous.

Par « nanomatériau », on entend au sens de la présente invention un matériau de dimension nanométrique, c'est-à-dire un matériau dont au moins une des dimensions est à l'échelle nanométrique. Autrement dit, il s'agit d'un matériau dont la taille nanométrique dans au moins une des dimensions de l'espace. Par exemple, la taille du matériau dans au moins une des dimensions de l'espace est comprise entre 1 et 500nm, de préférence entre 1 et 100 nm. Par exemple, il peut s'agir d'une couche mince.

Par « couche organo-minérale », on entend au sens de la présente invention une matrice comprenant au moins un composé minéral et au moins un composé organique. Ainsi, la couche organo-minérale peut être une couche sol-gel, par exemple de variété Silicate, Titanate, Zirconate, Stannate, Borate, Aluminate ou Yttriate, et comprenant au moins des nanocristaux organiques ou organo-métalliques.

La couche sol-gel peut en particulier être constituée majoritairement de silicate/oxyde de silicium amorphe, qui peut résulter de l'hydrolyse et polycondensation des alkoxydes de silicium inclus dans la solution initiale.

Cependant la couche sol-gel, ou le réseau polymérique, peut contenir également des radicaux carbonés alkyles, aryles et/ou aralkyles.

La couche inorganique et/ou organo-minérale peut tout particulièrement comprendre au moins une couche sol-gel.

Cette couche sol-gel peut particulièrement être une de variété silicate ou métalliques, en particulier accessible à partir de précurseurs alcoxydes de silicium, d'alcoxydes de titane ou d'autres métaux, en particulier choisis parmi Titane, Zirconium, Étain, Bore, Aluminium et Yttrium.

Selon la finalité de la matrice (balance hydrophile-hydrophobe, amélioration de la cristallinité, taille des nanocristaux, ...) les différents types d'alcoxydes pourront être utilisés à 100 % ou sous forme de mélange en proportions variables.

La chimie sol-gel et ses divers modes de mise en oeuvre sont connus de l'homme de métier, ont fait l'objet de nombreux rapports et publications, et ne seront pas développés dans le cadre du présent brevet. On citera par exemple, l'ouvrage de C.J. Brinker et G.W. Scherer, Sol-Gel Science, The Physics and Chemistry of SolGel Processing, Academic Press, New York, 1990 [ref 1]. On peut également citer d'autres articles de synthèse tels que D. Avnir, V.R. Kaufman and R. Reisfeld, J. Non. Cryst. Solids, 1985, 74, 395-406 [ref 2]; et C. Sanchez and F. Ribot, New J. Chem., 1994, 18, 1007-1047 [ref 3].

De manière générale, la couche inorganique et/ou organo-minérale comprenant une couche sol-gel peut être obtenue à partir d'un mélange sol-gel. Avantageusement, le mélange sol-gel contient au moins un composé organique ou organo-métallique fluorescent.Avantageusement, ce composé organique ou organo-métallique fluorescent initialement dissous dans le mélange sol-gel est cristallisé au sein de la matrice sol-gel pour former des nano-cristaux organiques ou organo-métalliques fluorescents. La cristallisation peut être effectuée par séchage du mélange sol-gel contenant le composé organique ou organo-métallique fluorescent. La vitesse du séchage peut être ajustée pour favoriser la formation de nano-cristaux.

On entend par mélange « sol-gel » au sens de la présente invention un mélange comprenant initialement au moins un alcoxyde métallique en présence d'eau, au moins un solvant organique et au moins un composé organique ou organo-métallique fluorescent, ledit mélange étant initialement sous la forme d'une solution et produisant un gel par réaction d'hydrolyse et de polycondensation. En effet, la présence d'eau permet d'initier des réactions d'hydrolyse et de condensation des alcoxydes métalliques formant un réseau inorganique conduisant à des gels puis des xérogels. Ces xérogels conduisent à un réseau solide inorganique structuré d'oxydes métalliques. La chimie sol-gel est par exemple citée dans le brevet français FR 2 853 307 [ref 4].

La couche sol-gel est susceptible d'être obtenue à partir de tout alcoxyde métallique connu de l'homme du métier compatible avec la chimie sol-gel.

Ainsi, dans un mode de réalisation, la couche sol-gel est susceptible d'être obtenue à partir d'un alcoxyde métallique de formule (I) :

R¹ₓM(OR²)_{y} (I)

dans laquelle :
- M est un métal choisi dans le groupe comprenant Si, Ti, Zr, Sn, B, Al et Y, par exemple Si.
- x est un nombre entier allant de 0 à 2, par exemple 0 ou 1 ;
- y est un nombre entier allant de 1 à 6, par exemple 3 ou 4 ;
- x+y correspond au nombre de coordination du métal M ;
- R¹ et R² représentent indépendamment un radical organique compatible avec la chimie sol-gel.

L'homme du métier saura sélectionner R¹ et R² à la lumière des ouvrages précités et de ses connaissances générales dans le domaine de la chimie sol-gel. Les exemples donnés ci-dessous sont donnés purement à titre illustratif et ne sont en aucun cas limitatifs.

Par exemple, R¹ et R² peuvent représenter indépendamment un radical carboné alkyle, aryle ou aralkyle.

Les radicaux alkyles peuvent être linéaires ou ramifiés, cycliques ou acycliques, saturés ou insaturés, et éventuellement comprendre ou porter des hétéroatomes, notamment O, N, S, B et P. Il peut par exemple s'agir de C₁₋₁₅alkyle; C₂₋₁₅alcenyle; C₂₋₁₅alcynyle; C₃₋₁₅cycloalkyle.

Les radicaux alkyles peuvent comprendre de 1 à 15 atomes de carbones, et notamment être choisis parmi les radicaux méthyle, éthyle, propyle, butyle, pentyle, hexyle, heptyle, octyle, nonyle, décyle, undécyle et dodécyle. Selon l'invention, le radical alkyle peut comprendre 1 à 15 atomes de carbone, par exemple 1 à 10 atomes de carbone, par exemple 1 à 6 atomes de carbone.

On notera que les radicaux alkyles peuvent comprendre ou porter un ou plusieurs hétéroatomes, notamment choisis dans le groupe comprenant l'oxygène, le soufre, l'azote, le phosphore et le bore. Autrement dit, les composés de formule (I) où R¹ et/ou R² est un radical alkyle englobent les composés de formule (I) où R¹ et/ou R² est un radical hétéroalkyle. Il peut par exemple s'agir d'un hétéroalkyle, d'un hétéroalcenyle, d'un hétéroalcynyle, d'un hétérocycle, d'un alkoxy, d'un alkylthio, d'une alkylamine, d'un alkylamide, d'une alkylimine, d'un alkylimide, d'un alkylester, d'un alkyléther, etc. Selon l'invention, le radical hétéroalkyle peut comprendre 1 à 15 atomes de carbone, par exemple 1 à 10 atomes de carbone, par exemple 1 à 6 atomes de carbone.

On entend par « aryle » au sens de la présente invention, un radical comprenant au moins un cycle satisfaisant la règle d'aromaticité de Hückel. Ledit aryle peut être mono- ou polycyclique, fusionné ou non. Les radicaux aryles peuvent comprendre de 4 à 15 atomes de carbone, en particulier de 6 à 10 atomes de carbone, et éventuellement comprendre des hétéroatomes, notamment O, N, S. Parmi les radicaux aryles, on peut citer les phényles, naphtyles et pyridinyles.

On notera que les radicaux aryles peuvent comprendre ou porter un ou plusieurs hétéroatomes, notamment choisis dans le groupe comprenant l'oxygène, le soufre, l'azote et le phosphore. Autrement dit, les composés de formule (I) où R¹ et/ou R² et un radical aryle englobent les composés de formule (I) où R¹ et/ou R² est un radical hétéroaryle, c'est-à-dire un groupe cyclique ou polycyclique hydrocarboné aromatique dans lequel au moins un atome de carbone est remplacé par un hétéroatome, notamment choisi dans le groupe comprenant l'oxygène, le soufre et l'azote.

On entend par « aralkyle » au sens de la présente invention, un radical alkyle substitué par un radical aryle, les termes « alkyle » et « aryle » étant tels que définis ci-dessus. Le point d'attachement du radical « aralkyle » au reste de la molécule (c'est-à-dire au métal M dans la formule (I)) est au niveau du groupe alkyle. Les radicaux aralkyles peuvent comprendre de 4 à 30 atomes de carbone, en particulier de 6 à 20 atomes de carbone, et éventuellement comprendre ou être substitués par des radicaux alkyles et/ou des hétéroatomes, notamment O, N, S et P. Parmi les radicaux aralkyles, on peut citer benzyle et tolyle.

Dans un mode de réalisation particulier, R¹ et R² peuvent représenter indépendamment un radical alkyle en C₁ à C₁₅, hétéroalkyle C₁ à C₁₅, aryle en C₆ à C₂₅, hétéroaryle en C₄ à C₂₅, ou aralkyle en C₆ à C₂₀; les radicaux R¹ et R² étant indépendamment éventuellement substitués par un ou plusieurs groupes R indépendamment choisis dans le groupe comprenant un radical alkyle en C₁ à C₁₀, hétéroalkyle C₁ à C₁₀; aryle en C₆ à C₁₀ ou hétéroaryle en C₄ à C₁₀ ; F ; Cl ; Br; I; -NO₂; -CN ; ou une fonction -GR^{G1} dans laquelle G est -O-, -S-, -NR^{G2}-, -C(=O)-, -C(=O)O-, -C(=O)NR^{G2}-, où chaque occurrence de R^{G1}, R^{G2} et R^{G3} est indépendamment des autres occurrences de R^{G1} un atome d'hydrogène, ou un radical alkyle en C₁ à C₁₀, hétéroalkyle C₁ à C₁₀; aryle en C₆ à C₁₀ ou hétéroaryle en C₄ à C₁₀ ; ou bien, lorsque G représente -NR^{G2}-, R^{G1} et R^{G2} conjointement avec l'atome d'azote auquel ils sont liés forment un hétérocycle ou un hétéroaryle éventuellement substitué.

Les radicaux alkyles, aryles et/ou aralkyles peuvent être liés directement aux atomes de silicium, titanium, zirconium, étain, bore, aluminium, yttrium, notamment par une liaison covalente. On peut alors parler de matériaux hybrides organo-mineraux, ainsi que par exemple les matériaux connus dans le domaine des lentilles de contact obtenues par sol-gel.

La couche sol-gel peut comprendre uniquement du silicium, ou du silicium et du Titane, du Zirconium et/ou de l'Étain. Cette couche sol-gel peut notamment être obtenue en utilisant d'autres types d'alkoxydes métalliques, comme des alkoxydes de Titane, Zirconium et/ou Étain.

Dès le mélange sol-gel constitué, les alcoxydes métalliques réagissent via des réactions d'hydrolyse et de condensation, conduisant à des « polymères inorganiques ». Dans le domaine de la chimie sol-gel, le terme « polymère inorganique » est utilisé par analogie avec les polymères organiques. A titre illustratif, les réactions suivantes d'hydrolyse (1) et de condensation (2) des alcoxydes métalliques peuvent intervenir.

(1) R¹ₓM(OR²)_{y} + H₂O → R¹ₓ(OR²)_{y-1}M(OH) + HOR²

(2) 2 R¹ₓ(OR²)_{y-1}M(OH) → R¹ₓ(OR²)_{y-1}M-O-M(OR²)_{y-1}R¹ₓ + H₂O

où R¹, R², x et y sont tels que définis précédemment.

Avantageusement, le mélange sol-gel du procédé de l'invention peut être homogène et stable chimiquement et mécaniquement. Par exemple il peut être stable pendant plusieurs mois voire plusieurs années ce qui présente un fort avantage pour la mise en oeuvre pratique de la présente invention.

On entend par « alcoxydes métalliques », également appelés « précurseurs sol-gel » au sens de la présente invention, tout composé métallique de formule (I). Selon l'invention, on peut utiliser tout alcoxyde métallique connu de l'homme du métier dans la mesure où l'on peut obtenir un réseau sol-gel polymérique interconnecté. L'homme du métier pourra notamment utiliser tout alcoxyde métallique propre à la chimie sol-gel connu. Pour cela, le lecteur peut notamment se référer au livre de référence C.J. Brinker et G.W. Scherer, Sol-Gel Science, The Physics and Chemistry of SolGel Processing, Academic Press, New York, 1990 [ref 1]. Voir également ref 2 et 3.

On entend par « solvant » au sens de la présente invention tout solvant connu de l'homme du métier compatible avec le procédé de mise en oeuvre de la présente invention, c'est-à-dire pouvant rendre miscibles les alcoxydes métalliques et l'eau d'hydrolyse afin d'obtenir des solutions homogènes, notamment propres à leur mise en forme en couche mince ou sous forme de film. Avantageusement, le solvant permet dissoudre totalement le composé fluorescent.

Le solvant peut être par exemple choisi dans le groupe comprenant les alcools R^{S}-OH où R^{S} est un radical alkyle en C₁ à C₆, les cétones R^{S1}-C(=O)-R^{S2}, les éthers R^{S1}-O-R^{S2}, où R^{S1} et R^{S2} sont indépendamment des radicaux alkyles en C₁ à C₆, le tétrahydrofurane, l'acétonitrile, le diméthylformamide, le toluène, le diméthylsulfoxyde, le dioxane, l'acétone, l'acide acétique, l'acide formique, le dichlorométhane, le chloroforme, le dichloroéthane, l'acétate d'éthyle, le diéthyléther ou un mélange de ceux-ci.

Selon l'invention, l'eau présente dans le mélange sol-gel peut être introduite initialement en faible quantité, celle-ci étant suffisante pour initier les réactions conduisant à la polycondensation du réseau inorganique, ou en quantité supérieure à la quantité initiale d'alcoxyde métallique. Par exemple le mélange sol-gel peut comprendre initialement un pourcentage molaire d'eau par rapport au nombre de fonctions alcoxydes (-OR²) de 10 à 400%, de préférence de 20 à 100%, de manière plus préférée de 50 à 100 %.

Les alcoxydes de silicium précurseurs de silicates peuvent être choisis, par exemple, dans le groupe comprenant le tétraméthoxysilane (TMOS, Si(OCH₃)₄), le tétraéthoxysilane (TEOS, Si(OC₂H₅)₄), le méthyltriméthoxysilane (MTMOS, CH₃Si(OCH₃)₃), le méthyltriéthoxysilane (MTEOS, CH₃Si(OC₂H₅)₃), l'éthyltriéthoxysilane (ETEOS, C₂H₅Si(OC₂H₅)₃), le 1,2-bis(triméthoxysilyl)ethane (TMSE), le 3-glycidoxypropyl)trimethoxysilane (GPTMS), ou un mélange de ceux-ci.

Pour les alcoxydes métalliques précurseurs de titanates, zirconates, stannates, borate, aluminates et yttriates, le lecteur pourra se référer par exemple à C.J. Brinker et G.W. Scherer, Sol-Gel Science, The Physics and Chemistry of SolGel Processing, Academic Press, New York, 1990 [ref 1]. Voir également ref 2 et 3.

Dans le cas d'une matrice obtenue par mélange équimoléculaire MTMOS et TMOS, les substituants méthyles non-liants de MTMOS peuvent couvrir, au moins en partie, les pores du gel et favoriser l'agrégation des nano-cristaux, tandis que les groupes silanols peuvent former des liaisons, notamment hydrogènes, avec les molécules organiques.

Tout particulièrement la couche inorganique et/ou organo-minérale, en particulier la couche sol-gel, se présente sous la forme d'un film.

Par « film », on peut entendre au sens de la présente invention une structure dans laquelle l'épaisseur est de l'ordre de quelques centaines de nanomètres ou de l'ordre du micromètre, et en particulier une épaisseur allant de 50 nm à 1500 nm, en particulier de 80 à 1200 nm, voire de 100 à 1000 nm.

Tout particulièrement, ce film présente une largeur et/ou une longueur de 1 mm à 10 cm, en particulier de 5 mm à 5 cm.

Les nano-cristaux peuvent être fluorescents, tout particulièrement ils absorbent et ré-émettent de la lumière dans les longueurs d'onde du visible ou du proche infra-rouge.

La luminescence peut se répandre sur un ensemble de molécules luminescentes, ou chromophores, par exemple allant de 10³ à 10¹⁰, notamment de 10³ à 10⁹, en particulier de 10⁴ à 10⁸, voire de 10⁵ à 10⁷ molécules par nano-cristal.

Tout particulièrement, les nano-cristaux émettent une fluorescence, qui est dispersée sur l'ensemble des fluorophores et qui est éteinte ou désactivée (« quenched ») simultanément. Par « simultanément », on entend de 0,001 à 10 ns, en particulier de 0,01 à 5 ns, en particulier de 0,1 à 2 ns, voire de 0,5 à 1,5 ns.

On entend par composé ou nanocristal « fluorescent » au sens de la présente invention, un composé ou un nanocristal ayant la propriété d'émettre, suite à une excitation lumineuse, un rayonnement électromagnétique dans le domaine de la lumière visible ou dans le proche infra-rouge (IR). Le signal lumineux d'excitation peut provenir d'une source lumineuse telle qu'un laser, une lampe à arc ou des diodes électroluminescentes (LED). Par exemple, le composé ou le nanocristal fluorescent peut émettre un rayonnement électromagnétique aux longueurs d'onde de 400 à 1200 nm, qui correspond à la fenêtre de relative transparence des tissus vivants. Par exemple, le composé ou le nanocristal fluorescent peut émettre un rayonnement électromagnétique aux longueurs d'onde de 400 à 1000 nm, par exemple de 550 à 800 nm, qui correspond à une fluorescence dans le rouge et le proche infra-rouge. Tout particulièrement, le composé ou le nanocristal fluorescent peut émettre une fluorescence dans le visible et proche infra-rouge.

Les nano-cristaux peuvent être des nano-cristaux organiques ou organo-métalliques, en particulier être fluorescents, notamment comprendre au moins un, voire être constitués d'un, composé fluorescent à l'état cristallin.

Les nano-cristaux peuvent comprendre au moins un composé molécule organique ou complexe de coordination qui fluoresce dans l'état solide et soluble dans un solvant organique.

Selon l'invention, le composé organique ou organo-métallique fluorescent peut être par exemple choisi dans le groupe comprenant les composés fluorescents de la famille des polyaromatiques, par exemple le diphénylanthracène, le rubrène ou le tétracène, la famille des aromatiques, par exemple le 4-(N,N-diethylamino)-b-nitrostyrene (DEANST), 1,4-di(2,5-phenyloxazole)benzene (POPOP) ou le N-4-nitrophenyl-(L)-prolinol (NPP), la famille des stilbènes, par exemple le cyano-méthoxy-nitro-stilbène (CMONS), le diéthyl-amino-nitro-stilbène ou le stilbène, la famille des naphtilimides, par exemple le naphtilimide, la famille des rhodamines, par exemple la rhodamine B, la famille des auramines, par exemple l'auramine O, la famille des pérylènes diimides, les dérivés du dipyrrométhène difluorure de bore, les complexes de terre rares, par exemple les complexes d'Europium.

Le composé fluorescent à l'état solide, voire cristallin peut en particulier être choisi dans le groupe comprenant le cyanométhoxynitrostilbène, le naphtilimide, des molécules polyaromatiques, comme le rubrène ou le tétracène, les perylènes diimides, le diphénylanthracène, les dérivés du dipyrrométhène difluorure de bore (Dipyrromethene Boron Difluoride - Bodipy®), et les complexes d'Europium.

Dans un mode de réalisation particulier, le composé fluorescent est choisi dans la famille des polyaromatiques, comme le rubrène, l'auramine O ou la rhodamine B, par exemple.

Les nano-cristaux peuvent être insérés dans les pores de la couche inorganique et/ou organo-minérale.

Par « pores », on entend au sens de la présente invention des interstices ou des vides entre les chaînes du réseau formant la couche inorganique et/ou organo-minérale (par exemple, le réseau silicate, titanate, zirconate, stannate, borate, aluminate, yttirate etc.) qui a polymerisé/polycondensé durant le procédé sol-gel.

Les nano-cristaux observés par microscopie électronique en transmission peuvent être de forme sphérique et peuvent présenter un diamètre allant de 10 à 500 nm, notamment de 100 à 400 nm, en particulier de 150 à 300 nm.

Les nano-cristaux peuvent présenter une distribution en taille assez homogène, en particulier variant au maximum de +/- 20 %, voire +/- 10 %.

Les nano-cristaux présents dans la couche inorganique et/ou organo-minérale présentent au moins une partie en contact direct avec l'extérieur, en particulier ils émergent de 2 à 50 % environ de leur diamètre par rapport à la couche inorganique et/ou organo-minérale, voire de 5 à 30 % de leur diamètre, en particulier lorsque les nanocristaux sont sphériques. La couche inorganique et/ou organo-minérale peut être une matrice sol-gel.

Les nanocristaux peuvent dépasser de la couche inorganique et/ou organo-minérale de 1 à 100 nm, en particulier de 2 à 80 nm, voire de 3 à 50 nm.

Il suffit que les nanocristaux émergent un peu en surface pour être en contact direct avec l'extérieur (solution bio) soit de quelques nm à quelques dizaines de nm.

Afin de présenter une résistance optimale, il est peut-être souhaitable de ne pas trop décaper la surface des couches sol-gel afin que les nanocristaux ne soient pas trop émergeants pour permettre une stabilisation mécanique excellente et ainsi éviter qu'ils se décrochent trop aisément de la couche sol-gel. Donc la hauteur d'émergence peut dépendre en partie du diamètre des nanocristaux.

Ainsi, selon un mode de réalisation particulier, la hauteur d'émergence ne dépasse pas le tiers du diamètre des nanocristaux, notamment afin qu'ils demeurent bien ancrés dans la couche sol-gel.

Cette mesure peut notamment être effectuée par microscopie à force atomique en mode oscillant intermittent en enregistrant la topographie de l'échantillon.

Avantageusement, les nano-cristaux présents dans la couche inorganique et/ou organo-minérale sont solidement ancrés dans celle-ci, en particulier ils peuvent résister à une immersion dans une solution biologique à analyser (brins d'ADN, protéines...) et cela pendant quelques minutes.

Par « résister », on entend que plus de 95 %, notamment plus de 99 %, voire plus de 99,9 %, des nanocristaux présents au début du test sont encore présents dans le (nano)matériau à la fin du test.

Dans un mode de réalisation particulier, ces nano-cristaux peuvent présenter à leur surface au moins un type de sonde. Les nano-cristaux peuvent présenter un ou plusieurs types de sonde identiques ou différents, par exemple un, deux, trois, quatre ou cinq sondes identiques ou différentes, en particulier un, deux, trois, quatre ou cinq sondes de types différents.

Au moins une sonde peut notamment être choisie dans le groupe comprenant les indicateurs colorés, l'ADN, les oligonucléotides ou polynucléotides, les polypeptides, les protéines, les anticorps, les sucres, les glycoprotéines et les lipides. Par exemple, les indicateurs colorés peuvent être sélectionnés parmi les indicateurs de pH, rédox et/ou complexation.

Cette sonde peut être simplement adsorbée ou bien greffée sur les nano-cristaux, en particulier via une liaison chimique. Par exemple, la sonde peut-être absorbée ou greffée via notamment une liaison covalente, ionique, électrostatique, ionocovalente, une liaison hydrogène, des interactions hydrophobes, les forces de van der waals.

La sonde peut être chimiquement modifiée avec une fonction chimique adéquate de manière à pouvoir se greffer à la surface du nano-cristal.

Avantageusement, la sonde éventuellement chimiquement modifiée ne se lie pas à la surface de la couche sol-gel, notamment elle est insensible aux sites M-OH libres présents à la surface de la couche sol-gel.

Ainsi, les nano-cristaux émergeants constituant, grâce à leur fluorescence, la fonction de signalisation, peuvent ainsi être fonctionnalisés par greffage d'une biomolécule portant une fonction sonde, tel demi-brin d'ADN, pour réaliser ainsi une bio-puce, capteur ou senseur comprenant un (nano)matériau selon l'invention, ou un support pour bio-puce, capteur ou senseur. Cet aspect de l'invention est discuté plus en détail dans les sections qui suivent.

Selon un autre de ses aspects, l'invention a pour objet un procédé de fabrication de (nano)matériau selon l'invention, en particulier comprenant une couche inorganique et/ou organo-minérale, dans laquelle est intégré au moins un type de nano-cristal fluorescent organique ou organo-métallique émergeant de la surface de ladite couche de sorte qu'au moins une partie est en contact direct avec le milieu extérieur comprenant au moins l'étape suivante :
a) dissolution d'une partie d'une couche inorganique et/ou organo-miriérale de manière à faire émerger au moins une partie des nano-cristaux de la couche inorganique et/ou organo-minérale ou à augmenter la surface des nano-cristaux en contact avec le milieu extérieur. En particulier, dans l'étape de dissolution, ladite couche inorganique et/ou organo-minérale comprend ou inclut au moins un type de nano-cristal fluorescent.

Cette étape de dissolution partielle de la couche sol-gel peut être effectuée par des agents, notamment chimiques, permettant de décaper de manière homogène la surface de la couche inorganique et/ou organo-minérale, en particulier sans détériorer les propriétés de fluorescence des nano-cristaux.

Tout particulièrement, cette étape est effectuée par des agents permettant de décaper, choisis parmi des solutions aqueuses basiques, en particulier des bases inorganiques ou organiques, fortes ou faibles, notamment de soude, d'ammoniaque et/ou de potasse. Tout particulièrement ces solutions basiques présentent une concentration en base allant de 10⁻⁶ à 10⁻¹ M, et notamment de 10⁻³ à 10⁻¹ M.

Ainsi, cette étape peut permettre un amincissement régulier de la couche inorganique et/ou organo-minérale, par décapage de la surface de la couche inorganique et/ou organo-minérale, et en particulier de matrices sol-gel silicatées.

Tout particulièrement cette étape de dissolution contrôlée permet une vitesse d'amincissement allant de 5 à 100 nm/h, voire 10 à 50 nm/h.

Le procédé selon l'invention peut également comprendre une étape consistant à préparer au moins une couche de matrice, en particulier de matrice sol-gel, comprenant ou incluant des nano-cristaux.

Ainsi, dans un mode de réalisation particulier, l'invention se rapporte à un procédé de fabrication de (nano)matériau comprenant une couche inorganique et/ou organo-minérale, dans laquelle est intégré au moins un type de nano-cristal fluorescent organique ou organo-métallique émergeant de la surface de ladite couche de sorte qu'au moins une partie est en contact direct avec le milieu extérieur, ledit procédé comprenant :
a) une étape consistant à préparer au moins une couche inorganique et/ou organo-minérale comprenant ou incluant des nano-cristaux fluorescents organiques ou organo-métalliques, et
b) une étape de dissolution d'une partie de ladite couche inorganique et/ou organo-minérale obtenue à l'étape a) de manière à faire émerger au moins une partie des nano-cristaux de la couche inorganique et/ou organo-minérale ou à augmenter la surface des nano-cristaux en contact avec le milieu extérieur.

Dans un mode de réalisation, la couche inorganique et/ou organo-minérale est une couche sol-gel. Ainsi, l'étape a) peut comprendre une étape préalable (0) de préparation d'un mélange sol-gel comprenant :
(i) une étape (0a) de préparation d'un mélange sol-gel initial, par exemple en mélangeant dans un solvant au moins un composé organique ou organo-métallique fluorescent et au moins un alcoxyde métallique, par exemple de formule (I), en présence d'eau ; et éventuellement,
(ii) une étape de stockage (0b) du mélange sol-gel initial pendant une durée d afin de laisser réagir le mélange initial.

Selon l'invention, la durée d peut être inférieure à plusieurs mois voire plusieurs années. Par exemple, la durée d peut être de 1 jour à 1 année, par exemple de 7 jours à 21 jours. En effet, le stockage du mélange permet de laisser vieillir le mélange afin de bien avancer les réactions d'hydrolyse et de condensation des alcoxydes métalliques.

Selon l'invention, l'étape (0) de préparation du mélange sol-gel peut comprendre l'ajout d'un acide au mélange initial. En effet, l'acide, en abaissant le pH du mélange, permet de favoriser l'obtention de longues chaînes inorganiques qui sont favorables à la formation d'une couche inorganique et/ou organo-minérale dense autour des cristaux organiques ou organo-métalliques. Selon l'invention, le pH du mélange peut être de 1 à 7, de préférence de 1 à 2.

On entend par « acide » au sens de la présente invention les acides de Brönsted, minéraux ou organiques. Parmi les acides utilisables, on peut citer par exemple l'acide chlorhydrique, l'acide nitrique ou l'acide acétique.

Le composé organique ou organo-métallique fluorescent, l'alcoxyde métallique et le solvant peuvent être choisis selon l'un quelconque des modes de réalisation décrits ci-dessus pour la mise en oeuvre des nanomatériaux de l'invention.

L'étape a) peut notamment être effectuée par la cristallisation de nano-cristaux organiques ou organo-métalliques fluorescents dans des couches minces de matrice sol-gel. Tout particulièrement cette étape peut être réalisée par centrifugation « à la tournette » (« spin-coating »).

Tout particulièrement, l'élaboration de cette couche inorganique et/ou organo-minérale, éventuellement sous forme de couche mince, peut être réalisée à température ambiante par hydrolyse et condensation d'une solution comprenant des précurseurs de la couche inorganique et/ou organo-minérale, notamment sol-gel, comme des alcoxydes de silicium, dans laquelle les molécules organiques ou organo-métalliques sont dissoutes dans un solvant, notamment organique.

Par « organo-métallique », on entend des composés comprenant une partie organique et une partie métallique.

La nano-cristallisation peut résulter d'une forte nucléation instantanée, suivie d'un contrôle de la croissance des nuclei par la viscosité et/ou la porosité du gel. La sursaturation de la phase organique peut être provoquée par l'évaporation très rapide du solvant organique.

En particulier dans la procédure centrifugation à la tournette (« spin-coating »), selon la vitesse de rotation, allant par exemple de 1000 à 4000 tr/mn, la viscosité du solvant et ou le temps de dépôt, des couches minces et/ou transparentes, par exemple de 100 à 1000 nm peuvent être réalisées.

La couche inorganique et/ou organo-minérale de l'étape a) peut ensuite subir une étape de recuit, notamment à des températures voisines du point de fusion des nano-cristaux organiques. Cette étape de recuit est destinée à éliminer toute trace de solvant et stabiliser davantage la couche sol-gel, notamment inorganique (élimination de solvant résiduel, densification de la matrice). L'étape de recuit peut être conduite en particulier à des températures allant de 50 à 250°C, notamment de 80 à 150°C.

Par « température voisine du point de fusion », on entend au sens de la présente invention une température comprise dans une gamme allant de point de fusion moins 5°C à point de fusion moins 100°C, en particulier de point de fusion moins 10°C à point de fusion moins 50°C.

Cette étape de recuit peut permettre également d'améliorer la cristallinité des agrégats et/ou la stabilisation de la couche inorganique et/ou organo-minérale.

La taille des nano-cristaux peut ainsi être ajustée selon les conditions de nano-cristallisation confinée dans la couche inorganique et/ou organo-minérale, notamment dans la matrice sol-gel, en particulier silicatée.

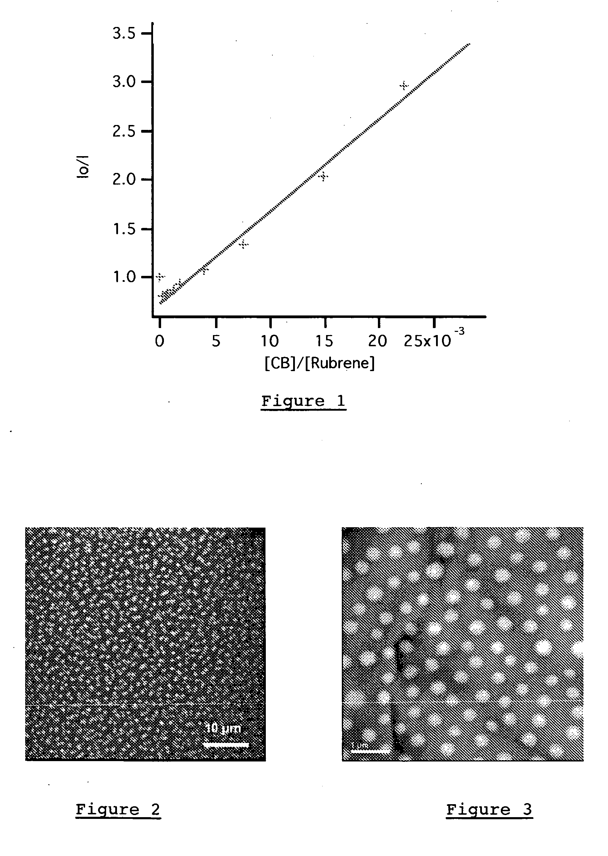

Dans la mesure où les nano-cristaux sont fluorescents, ils peuvent être visualisés grâce à des techniques comme la microscopie photonique confocale. Ceci est illustré par la figure 2 qui est une image par microscopie photonique confocale de nano-cristaux de rubrène en matrice sol-gel.

Dans le cas où les nano-cristaux présentent une taille inférieure à 100 nm, la microscopie électronique en transmission peut être utilisée pour observer la distribution et la taille des nano-cristaux. Ceci est illustré par la Figure 3 qui est une image par microscopie électronique en transmission de nano-cristaux de rubrène en matrice sol-gel.

Ces visualisations directes des nano-cristaux peuvent permettre d'ajuster les paramètres influant sur la cristallisation confinée des chromophores (molécules organiques ou organo-métalliques fluorescentes), en particulier sur la nature des précurseurs permettant de former la matrice sol-gel, par exemple la nature des alcoxydes de silicium, les cinétiques d'hydrolyse et de condensation, la nature et les taux de solvants, la concentration en fluorophores, la sursaturation et les paramètres de centrifugation.

Selon un autre de ses objets, la présente invention concerne un film de (nano)matériau selon l'invention. Dans un mode de réalisation particulier, il s'agit d'un film comprenant une matrice sol-gel comprenant des nano-cristaux stabilisés mécaniquement, lesdits nano-cristaux émergeant de la surface dudit film de sorte qu'au moins une partie de chaque nano-cristal est en contact direct avec le milieu extérieur. En particulier le (nano)matériau est une matrice sol-gel comprenant des nano-cristaux stabilisés mécaniquement, lesdits nano-cristaux émergeant de la surface dudit film de sorte qu'au moins une partie de chaque nano-cristal est en contact direct avec le milieu extérieur, disposé sur un support solide, de type lame de verre par exemple.

Les techniques de préparation de tels films sont connues de l'homme du métier, et ne seront pas développées dans la présente demande. Dans ce contexte, plusieurs méthodes de dépôt de couches minces sol-gel peuvent être utilisées : techniques de centrifugation du substrat (plusieurs milliers de tours par minute). On peut également utiliser d'autres techniques telles que le tirage du substrat verticalement (« dip-coating » en anglais). Le lecteur pourra se référer à l'ouvrage de Brinker et Scherer précité [Ref 1].

Comme discuté auparavant, les dispositifs basés sur la détection d'un signal fluorescent sont notamment utilisés dans les domaines reposant sur la détection de réactions chimiques ou biologiques (par exemple les biopuces).

Les biopuces sont un outil biochimique de collection massive d'information notamment sur les acides nucléiques (puces ADN) et les acides aminés (puces à protéines), les antigènes et anticorps (capteurs immunologiques). Associées à des techniques de traitement numérique des informations récoltées, les puces ADN permettent de mener les recherches (détection, séparation, identification, étude) permettant d'accéder directement aux ADN.

Les puces à protéines permettent de détecter, identifier, séparer, étudier les protéines et déterminer leurs activités, fonctions, interactions, modifications au cours du temps.

Les capteurs immunologiques basés sur une liaison avec une enzyme permettent de détecter des anticorps/antigènes.

D'une manière générale, un domaine d'application de l'invention concerne donc les puces, notamment les biopuces, capteurs et senseurs, qui se présentent sous forme d'un support solide à la surface duquel des éléments biochimiques sont immobilisés.

Ainsi, la présente invention trouve notamment des applications dans la fabrication de supports pour les biopuces capteurs ou senseurs. Il s'agit notamment de supports biopuces compatibles avec le greffage d'acides nucléiques (biopuces à ADN, biopuces à ARN), d'acides aminés (puces à protéines, puces immunologiques), ainsi que les biopuces cellulaires utilisées notamment dans les études de transfection.

Ainsi, selon un de ses aspects, l'invention concerne un support pour biopuce, capteur ou senseurs comprenant un substrat dont une surface comprend une couche de (nano)matériau ou de film selon l'invention. Dans un mode de réalisation, le (nano)matériau comprend au moins une couche inorganique et/ou organo-minérale, dans laquelle est intégré au moins un type de nano-cristal fluorescent organique ou organo-métallique émergeant de la surface de ladite couche de sorte qu'au moins une partie du nano-cristal est en contact direct avec le milieu extérieur.

Dans un mode de réalisation, le support comprend un substrat sur lequel le (nano)matériau de la présente invention est déposé, notamment sous forme de couche mince ou de film. Il peut s'agir de tout matériau solide connu de l'homme du métier, tel que par exemple les matériaux supports utilisés pour la fabrication des microsystèmes d'analyse et des biopuces. Il peut s'agir d'un substrat organique ou inorganique. Il peut être constitué par exemple d'un matériau choisi dans le groupe comprenant du verre, de la silice, du polycarbonate, du nymon, du polyméthylméthacrylate (PMMA), du polystyrène, du copolymère cyclooléfine (COC), et du polystyrène acrylonitrile (SAN). Le support est typiquement une lame de verre de taille comparable à une lame de microscope.

Le substrat peut être préalablement nettoyé afin d'améliorer l'adhérence de la couche de (nano)matériau à sa surface. Ce nettoyage peut être par exemple un nettoyage chimique, éventuellement suivi d'un traitement thermique. Les techniques de nettoyage en particulier celles utilisées pour la préparation de supports de biopuce, capteur et senseur, connues de l'homme du métier sont utilisables. Pour ce nettoyage, on peut utiliser tout solvant approprié pour dépoussiérer et/ou dégraisser la surface du substrat, de préférence sans l'abîmer. On peut citer par exemple comme solvant de nettoyage le trichloréthylène, l'acétone, l'alcool éthylique, l'eau désionisée, des solutions acides ou basiques, etc. Le nettoyage peut consister à baigner le substrat dans un ou plusieurs de ces solvants successivement. Le nettoyage est alors généralement suivi d'un séchage. Le nettoyage peut également consister tout simplement à débarrasser le substrat de ses poussières par un jet d'air comprimé.

La couche de (nano)matériau peut être déposée sur la surface du substrat par toute technique connue de l'homme du métier pour déposer ce type de matériau sur une surface. Tout particulièrement, lorsque le (nano)matériau comprend une couche sol-gel, cette étape peut être réalisée par :
(i) une étape (0a) de préparation d'un mélange initial, par exemple en mélangeant dans un solvant au moins un composé organique ou organo-métallique fluorescent et au moins un alcoxyde métallique, par exemple de formule (I), en présence d'eau ; éventuellement,
(ii) une étape de stockage (0b) du mélange initial pendant une durée d afin de laisser réagir le mélange initial, et
(iii) une étape de dépôt du mélange sol-gel obtenu à l'étape (ii) sur un support, et
(iv) une étape de centrifugation « à la tournette » (« spin-coating ») pour obtenir une couche inorganique et/ou organo-minérale comprenant des nano-cristaux organique sou organo-métalliques fluorescents à la surface du support.

En effet, un mélange sol-gel étant préparé par hydrolyse et condensation de sels ou d'alcoxydes de métaux ou de métalloides M, avantageusement en présence d'un solvant organique, ce solvant organique peut faciliter le dépôt et le séchage de la couche de (nano)matériau sur le substrat.

Dans un mode de réalisation, la couche de (nano)matériau selon l'invention peut être déposée sur ladite surface du substrat à une épaisseur de 50 nm à 1000 nm, voire 100 nm à 1000 nm. En fait, on comprend aisément que l'épaisseur minimale est une épaisseur qui permet avantageusement de couvrir la surface du substrat, sans laisser de trous. Quant à l'épaisseur maximale, il est de façon générale préférable de ne pas aller au-delà du micromètre. En effet, si l'épaisseur est supérieure au micromètre, des problèmes de craquage/fissuration de la couche liés à l'évaporation du solvant peuvent survenir (tensions internes dues aux forces capillaires ; voir ouvrage de référence de Brinker et Scherer [ref 1]). il est toutefois superflu d'utiliser des quantités trop importantes de (nano)matériau pour mettre en oeuvre la présente invention.

Une fois la couche inorganique et/ou organo-minérale comprenant des nano-cristaux organiques ou organo-métalliques fluorescents déposés sur le support, celui-ci peut être en outre soumis à une étape de dissolution d'une partie d'une couche inorganique et/ou organo-minérale de manière à faire émerger au moins une partie des nano-cristaux de la couche inorganique et/ou organo-minérale ou à augmenter la surface des nano-cristaux en contact avec le milieu extérieur.

Les divers modes de réalisation décrits ci-dessus pour la préparation du (nano)matériau selon l'invention sont applicables à la mise en oeuvre des supports pour puces, capteurs ou senseurs (e.g., solvants, composés fluorescents, alcoxydes métalliques, etc.).

En particulier, un ou plusieurs types de sonde identiques ou différents peuvent être greffés ou adsorbées sur la partie des nano-cristaux émergeant de la surface de la couche inorganique et/ou organo-minérale. Pour ce faire, on pourra appliquer les divers modes de réalisation décrits précédemment pour le (nano)matériau de l'invention (i.e., types de sonde, modes de liaison, etc..).

Dans un mode de réalisation particulier, le support selon l'invention peut comprendre un empilement de couches minces diélectriques formant un miroir de Bragg intercalé entre le substrat et la couche de nanomatériau. Pour la mise en oeuvre de cet aspect de l'invention, le lecteur pourra se référer aux documents brevet de la société Genewave, par exemple WO 2004/042376 [Ref 5] et WO 2007/045755 [Ref 6]. Il s'agit de substrats présentant en surface de réseau un miroir de Bragg pour aire en sorte que le signal fluorescent soit complètement réfléchi vers le détecteur pour augmenter la sensibilité du système. Ces substrats sont communément appelés « amplislides ».

Cette configuration particulière peut permettre d'augmenter le champ excitateur à l'intérieur de la couche de nanomatériau. Ainsi, cette configuration a pour effet d'augmenter l'excitation des nano-cristaux organiques ou organo-métalliques luminescents ancrés dans la couche de nanomatériau, et donc d'augmenter la quantité de lumière émise dans le substrat. On précise qu'un miroir de Bragg est un empilement successif de plusieurs couches minces diélectriques d'indices de réfraction n₁ et n₂ différents. L'épaisseur de chacune de ces couches est égale à λ/(4n), avec n pouvant prendre la valeur n₁ ou n₂. La variable 1 correspond à la longueur d'onde à laquelle on souhaite avoir une réflexion maximale pour le miroir de Bragg.

Selon un autre de ses aspects, l'invention se rapporte à l'utilisation du (nano)matériau selon l'invention pour la fabrication de supports pour puces, notamment des supports pour biopuces, capteurs ou senseurs.

Selon encore un autre de ses aspects, l'invention se rapporte à une puce, notamment une bio-puce, un capteur ou un senseur comprenant au moins un (nano)matériau ou un film selon l'invention. Les puces, notamment les biopuces, capteurs et senseurs et leurs divers modes de mise en oeuvre sont connus de l'homme de métier, ont fait l'objet de nombreux rapports et publications. À titre illustratif, on pourra se référer à (i) D. L. Gerhold, « Better Therapeutics through microarrays, Nature Genetics, 32, p 547-552 (2002) [Ref 7] ; (ii) M.A. Shipp « Diffuse large B-cell lymphoma outcome prediction by gene-expression profiling and supervised machine learning » Nature Medecine 8 pp 68-74 (2002) [Ref 8] ; and (iii) L. Quijada, M. Soto and J. M. Requena « Genomic DNA microarrays as a tool of gene expression in leishmania » Expression Parasitology, 111, pp 64-70 (2005) [Ref 9].

S'agissant d'une puce ADN par exemple, on peut greffer ou adsorber un ou plusieurs oligonucléotides ou polynucléotides sur les nano-cristaux organiques ou organo-métalliques luminescents émergeant de la surface de la couche inorganique et/ou organo-minérale du (nano)matériau selon l'invention. Avantageusement, ces oligonucléotides ou polynucléotides sont des oligonucléotides ou polynucléotides simples brins spécifiques connus. Leur rôle est de détecter des cibles marquées complémentaires, présentes dans le mélange complexe à analyser. En effet, le principe de détection utilisé dans les puces ADN repose sur les possibilités d'appariement de brins d'ADN avec leurs bases complémentaires. Ces oligonucléotides ou polynucléotides sont autant de sondes qui permettent d'hybrider des séquences d'ADN complémentaires provenant d'un échantillon biologique à analyser. Avantageusement, les nano-cristaux organiques ou organo-métalliques luminescents sont fluorescents. Une fois la puce hybridée, la détection des chaînes est effectuée par mesure du signal fluorescent correspondant. Les images obtenues sont numérisées pour être ensuite traitées par des algorithmes de traitement spécifiques de ces données, mis en oeuvre par des moyens informatiques.

Les divers modes de réalisation décrits ci-dessus pour la préparation du (nano)matériau et supports pour puces, capteurs et senseurs selon l'invention sont applicables à la mise en oeuvre des puces, capteurs ou senseurs (e.g., solvants, composés fluorescents, alcoxydes métalliques, etc.).

En particulier, un ou plusieurs types de sonde identiques ou différents peuvent être greffés ou adsorbés sur la partie des nano-cristaux émergeant de la surface de la couche inorganique et/ou organo-minérale. Pour ce faire, on pourra appliquer les divers modes de réalisation décrits précédemment pour le (nano)matériau de l'invention (i.e., types de sonde, modes de liaison, etc...).

Le (nano)matériau selon l'invention peut permettre d'obtenir une meilleure sensibilité (capacité de détection), et par exemple permettre de gagner jusqu'à 2 a 3 ordres de grandeur par rapport aux biopuces actuelles.

Ce gain en sensibilité peut notamment être dû à la conjugaison de deux facteurs principaux :
chaque nano-cristal peut être composé d'un très grand nombre de fluorophores (de 10⁴ à 10¹⁰ fluorophores par nano-cristal selon son diamètre), et
au transfert d'énergie donneur-donneur entre fluorophores, ainsi l'excitation lumineuse peut explorer un large domaine sur une distance de 8 nm en moyenne, soit deux fois le rayon de Förster, distance de transfert d'énergie entre un donneur (fluorophore isolé) et un accepteur d'énergie.

Ainsi, la sensibilité peut être améliorée d'un facteur 100 ou plus par rapport à un fluorophore isolé.

Cette amélioration peut être constatée en mesurant le nombre de mole de fluorophores éteint dans une suspension aqueuse de nanocristaux par mole d'inhibiteur de fluorescence. L'effet d'inhibition observé (modification du déclin de fluorescence) est beaucoup plus important dans le cas des nano-cristaux.

Tout particulièrement les puces à fluorescence selon l'invention peuvent présenter une capacité de détection (sensibilité) d'une molécule cible pour cent molécules fluorescentes. Cette capacité de détection, ou sensibilité, se mesure mieux au moyen de cristaux en solution où les concentrations sont mieux connues. Un exemple de ce mode de réalisation est donné dans l'Exemple 4 ci-dessous.

### Brève description des figures

La figure 1 représente un exemple de tracé de « Stern Volmer » de Io/I dont la pente donne le nombre de molécules de rubrène dans la nanoparticule éteintes par chaque molécule de quencheur, le bleu de cibacron (CB) par exemple. Autrement dit, il s'agit du nombre moyen de molécules de rubrène par nano-cristal organique fluorescent qui sont éteintes par chaque molécule de quencheur.

La figure 2 représente un exemple d'image par microscopie photonique confocale de nano-cristaux de rubrène en matrice sol-gel issue de précurseur TMOS/TMSE.

La figure 3 représente un exemple d'image par microscopie électronique en transmission de nano-cristaux de rubrène en matrice sol-gel issue de précurseur TMOS/TMSE

La figure 4 représente un exemple d'images enregistrées au microscope à force atomique (AFM) en mode intermittent montrant des nanocristaux de rubrène émergeant d'environ 35 nm de la surface d'une couche mince sol-gel issue de précurseur TMOS/TMSE.

La figure 5 représente un exemple d'images enregistrées au microscope à force atomique en champ proche (AFM) montrant des nanocristaux de rhodamine B émergeant de 2 à 3 nm de la surface d'une couche mince sol-gel silicatée issue de précurseur TMOS/GPTMS.

La figure 6 représente un exemple d'images enregistrées au microscope à force atomique en champ proche (AFM) montrant des nanocristaux d'auramine O émergeant de 15 à 20 nm de la surface d'une couche mince sol-gel silicatée issue de précurseur TMOS/TMSE.

### EXEMPLES

Le procédé de dissolution partielle de la surface des couches sol-gel pour rendre émergeant les nanocristaux organiques selon l'invention a été appliqué sur différentes couches nanocomposites afin d'illustrer l'aspect générique de l'invention. Des décapages ont été mis en oeuvre sur trois couches sol-gel différentes contenant des nanocristaux organiques différents. Ceux-ci sont détaillés dans les Exemples 1 à 3 ci-dessous.

**Exemple 1. NANOCRISTAUX DE RUBRENE EN COUCHES MINCES SOL-GEL SILICATEES ISSUES DE PRECURSEUR TMOS + TMSE**

La poudre microcristalline de rubrène (1,8 mg ; 0,033 mmoles) a été dissoute dans une solution contenant 4,570 mL de tétrahydrofurane, 0,257 mL de tétraméthylsiloxane (TMOS), 0,222 mL de 1,2-bis(triméthoxysilyl)ethane (TMSE) et 0,219 mL d'une solution HCl 0,1 M. Les proportions molaires dans le mélange initial sont : 2 TMOS + 1 TMSE + 48 THF + 10 H₂O + 0.005 rubrène. (H₂O se réfère à la quantité d'eau introduite avec la solution HCl 0,1 M).

La solution a ensuite été stockée pendant plusieurs jours afin de bien avancer les réactions d'hydrolyse et de condensation des alcoxydes. On note que le mélange sol-gel est très stable. On peut donc éventuellement laisser le mélange en stockage pendant plusieurs semaines, voire plusieurs mois si on le souhaite. Cependant, plusieurs jours sont suffisants pour bien faire hydrolyse et condensation des alcoxydes de départ.

Un volume de 200µL du mélange ainsi obtenu est déposé sur une lame de verre de microscope.

La lame de microscope est ensuite introduite dans un Spin coater de la société Suss Microtech de type RC8 GYRSET. La couche mince sol-gel est obtenue par spin-coating en utilisant les conditions suivantes :

vitesse de rotation : 4000 tr/min

accélération : 3000 tr/min²

durée de la rotation : 10s

La couche mince ainsi obtenue est ensuite recuite à 100°C entre 24h et 72h.

On obtient ainsi des nanocristaux de rubrène de 200 nm de diamètre moyen inclus dans une couche mince de 250 nm d'épaisseur. Les nanocristaux ont été visualisés par microscopie optique confocale (figure 2) et microscopie électronique en transmission (figure 3).

La couche mince nanocomposites incrustées de nanocristaux de rubrène est ensuite soumise à des conditions de dissolutions contrôlées. Ainsi, la couche mince est décapée pour faire apparaître les nanocristaux de rubrène en surface en la trempant dans une solution de NaOH diluée (10⁻³M) durant 16H.

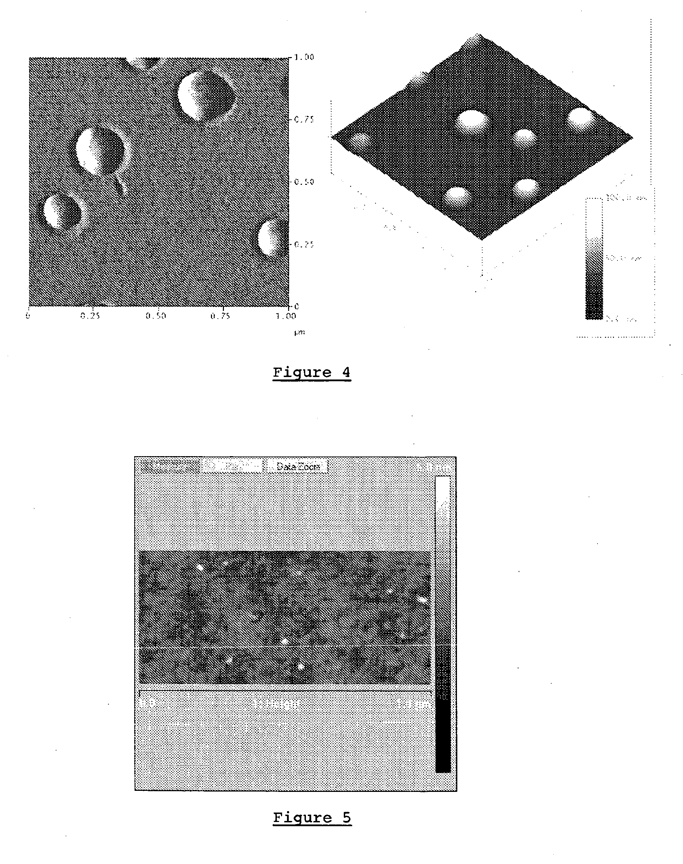

Les nanocristaux émergeant à la surface de la couche sol-gel sont présentés en figure 4. Cette figure permet de visualiser directement une lame supportant une couche sol-gel de très faible rugosité (0,5 nm RMS) qui supportent des nanocristaux émergeants qui sont la fonction de signalisation de ce nouveau type de détecteur.

Les images de la figure 4 ont été enregistrées au microscope à force atomique (AFM) en mode intermittent montrant des nanocristaux de rubrène émergeant à la surface de la couche mince sol-gel. Ces images montrent que les nanocristaux sont bien en contact avec le milieu extérieur mais qu'ils restent bien ancrés dans la couche. Il sont décapés à moins de 50% de leur diamètre, et de l'ordre de 30% au maximum. Les nanocristaux émergent d'environ 35 nm au-dessus de la couche sol-gel. On n'observe pas de perte (trou) de nanocristaux sur ces couches décapées qui sont ainsi prêtes à être fonctionnalisées.

Ces échantillons constituent un exemple de réalisation de base (fonction de signalisation de ce nouveau type de biopuce). Ainsi, nous avons pu maîtriser par dissolution lente (2-3 nm/h) le décapage de la surface de nos couches sol-gel permettant l'émergence des nanocristaux organiques tout en conservant la même rugosité d'environ 0.5 nm en RMS au niveau de la couche silicatée. Cette conservation de la très faible rugosité de la couche sol-gel illustre bien le parfait contrôle du décapage qui est très homogène en épaisseur sur toute la couche selon un procédé de dissolution par monocouches moléculaire, ce qui est tout à fait remarquable.

**Exemple 2. NANOCRISTAUX DE RHODAMINE B EN COUCHES MINCES SOL-GEL SILICATEES ISSUES DE PRECURSEUR TMOS/GPTMS**

Un mélange sol-gel a été préparé selon un mode opératoire similaire à celui de l'Exemple 1, en utilisant 21,7 mg de poudre microcristalline de rhodamine B 3,662 mL de MeOH, 0,267 mL de tétraméthylsiloxane (TMOS), 0,599 mL de 3-glycidoxypropyl)trimethoxysilane (GPTMS) et 0,277 mL d'une solution HCl 0,1 M. Les proportions molaires dans le mélange initial sont : 0.4 TMOS + 0.6 GPTMS + 20 MeOH + 8 H₂O + 0.01 rhodamine B. (H₂O se réfère à la quantité d'eau introduite avec la solution HCl 0,1 M).

Le mélange ainsi obtenu a été stocké pendant plusieurs jours afin de bien avancer les réactions d'hydrolyse et de condensation des alcoxydes. On note que le mélange sol-gel est très stable. On peut donc éventuellement laisser le mélange en stockage pendant plusieurs semaines, voire plusieurs mois si on le souhaite. Cependant, plusieurs jours sont suffisants pour bien faire hydrolyse et condensation des alcoxydes de départ.

Une couche mince sol-gel a été réalisée sur lame de microscope par spin-coating dans des conditions de spin coating et de recuit identiques à celles de l'Exemple 1.

On obtient ainsi des nanocristaux de rhodamine B de 40 nm de diamètre moyen inclus dans une couche mince silicatée de 180 nm d'épaisseur.

La lame est ensuite plongée dans une solution de NaOH à 1.10⁻³ mol/L pendant 4h.

Les nanocristaux émergent, après décapage de 2 à 3 nm (voir Figure 5).

La vitesse de dissolution pour cette matrice et avec NaOH à 1.10⁻³ mol/L est d'environ 2 nm par heure confirmant les résultats de l'exemple précédent.

**Exemple 3. NANOCRISTAUX D'AURAMINE O EN COUCHES MINCES SOL-GEL SILICATEES ISSUES DE PRECURSEUR TMOS/TMSE**

Un mélange sol-gel a été préparé selon un mode opératoire similaire à celui de l'Exemple 1, en utilisant 42,36 mg de poudre microcristalline d'auramine O 3,528 mL de THF, 0,257 mL de tétraméthylsiloxane (TMOS), 0,222 mL de 1,2-bis(triméthoxysilyl)ethane (TMSE) et 0,219 mL d'une solution HCl 0,1 M. Les proportions molaires dans le mélange initial sont : 2 TMOS + 1 TMSE + 75 THF + 10 H₂O + 0.04 auramine O. (H₂O se réfère à la quantité d'eau introduite avec la solution HCl 0,1 M).

Le mélange ainsi obtenu a été stocké pendant plusieurs jours (3 ou 4 jours) afin de bien avancer les réactions d'hydrolyse et de condensation des alcoxydes. Comme dans les exemples précédents, le mélange sol-gel est stable. On peut donc éventuellement laisser le mélange en stockage pendant plusieurs semaines, voire plusieurs mois si on le souhaite. Cependant, plusieurs jours sont suffisants pour bien faire hydrolyse et condensation des alcoxydes de départ.

Une couche mince sol-gel a été réalisée sur lame de microscope par spin-coating dans des conditions de spin coating et de recuit identiques à celles des exemples précédents.

On obtient ainsi des nanocristaux d'auramine O de 200 à 300 nm de diamètre moyen inclus dans une couche mince silicatée de 300 nm d'épaisseur.

La lame est ensuite plongée dans une solution de NaOH à 1.10⁻¹mol/L pendant 1H30. On note que la méthode de décapage fonctionne également avec une solution nettement plus concentrée que celles utilisées dans les exemples précédents. La vitesse de dissolution s'en trouve plus élevée.

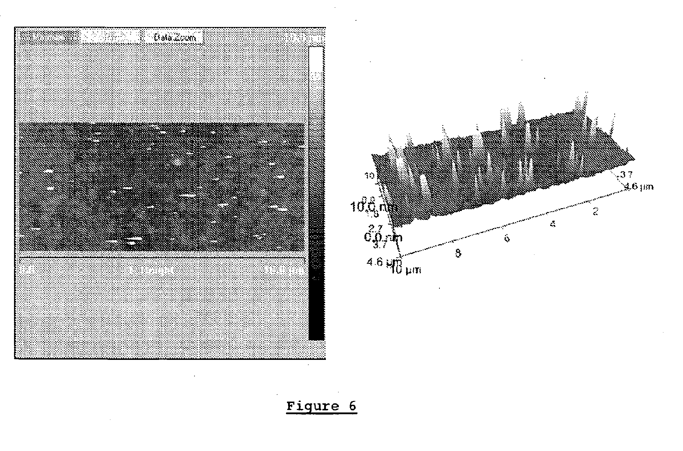

Après décapage, Les nanocristaux émergent de 15 à 20 nm (voir Figure 6). La vitesse de dissolution pour une telle matrice et pour [NaOH] = 1.10⁻¹mol/L est d'environ 8-10 nm par heure.

Les trois exemples précédents démontrent l'applicabilité de l'invention à diverses couches sol-gel, et différents types de nano-cristaux, de tailles différentes

### Exemple 4

Une suspension aqueuse de nanocristaux est préparée. 10 mL d'une solution de rubrène à 1,3 mmol/L dans un mélange l'éthanol/THF 3 :7 est injectée avec une seringue avec une petite aiguille dans 100 mL d'une solution aqueuse de CTACl (chlorure de cethyl trimethyl ammonium) à 5 mmol/L. La solution est filtrée à travers un filtre millipore de taille coupure de 50 nm. La concentration en molécule fluorescente est mesurée par absorption dans une cuvette en quartz de 1 cm de trajet optique. La fluorescence est mesurée dans un spectrofluorimètre SPEX Fluorolog avec une excitation à 495 nm avec des fentes de 2 nm à l'émission et à l'excitation. On mesure l'intensité de fluorescence pour des ajouts connus de « quencheur » (molécule sonde qui inhibe la fluorescence des nanocristaux par transfert d'énergie non radiatif entre le nano-cristal initialement excité et cette molécule se trouvant en surface du nano-cristal). Un quencheur typique est le bleu de cibachron. Un autre est le bleu de méthylène. Tous deux sont dissous à 1 mmol/L dans l'eau. On montre sur la figure 1 qu'avec un quencheur pour 100 molécules de rubrène on a déjà 33% d'extinction de la fluorescence : Le tracé de "Stern Volmer" de Io/I est une droite en accord avec une adsorption du quencheur, le bleu de cibacron (CB), sur les nanocristaux de rubrène dans cet exemple. La pente donne le nombre de molécule de rubrène dans la nanoparticule dont la fluorescence est éteinte par chaque molécule de quencheur.

### Liste des références

(1) C.J. Brinker et G.W. Scherer, Sol-Gel Science, The Physics and Chemistry of SolGel Processing, Academic Press, New York, 1990.
(2) D. Avnir, V.R. Kaufman and R. Reisfeld, J. Non. Cryst. Solids, 1985, 74, 395-406.
(3) C. Sanchez and F. Ribot, New J. Chem., 1994, 18, 1007-1047.
(4) Brevet français FR 2 853 307.
(5) WO 2004/042376.
(6) WO 2007/045755.
(7) D. L. Gerhold, « Better Therapeutics through microarrays, Nature Genetics, 32, p 547-552 (2002).
(8) M.A. Shipp « Diffuse large B-cell lymphoma outcome prediction by gene-expression profiling and supervised machine learning » Nature Medecine 8 pp 68-74 (2002).
(9) L. Quijada, M. Soto and J. M. Requena « Genomic DNA microarrays as a tool of gene expression in leishmania » Expression Parasitology, 111, pp 64-70 (2005).

## Revendications

1. Matériau comprenant au moins une couche inorganique et/ou organo-minérale, dans laquelle est intégré au moins un type de nano-cristal fluorescent organique ou organo-métallique émergeant de la surface de ladite couche de sorte qu'au moins une partie du nano-cristal est en contact direct avec le milieu extérieur, dans lequel ledit au moins un type de nano-cristal émerge de 2 à 50 % de son diamètre par rapport à la couche inorganique et/ou organo-minérale.

2. Matériau selon la revendication 1 dans lequel la couche inorganique et/ou organo-minérale est une matrice sol-gel de variété Silicate, Titanate, Zirconate, Stannate, Borate, Aluminate ou Yttriate.

3. Matériau selon la revendication 1 ou 2 se présentant sous forme de film.

4. Matériau selon l'une quelconque des revendications 1 à 3 dans lequel ledit au moins nano-cristal comprend au moins une molécule organique ou un complexe de coordination qui fluoresce dans l'état solide et qui est soluble dans un solvant organique, la molécule organique ou complexe de coordination étant choisie dans le groupe comprenant les composés fluorescents de la famille des polyaromatiques, la famille des aromatiques, la famille des stilbènes, la famille des naphtilimides, la famille des rhodamines, la famille des auramines, la famille des pérylènes diimides, les dérivés du dipyrrométhène difluorure de bore, et les complexes de terre rares.

5. Matériau selon la revendication 4 dans lequel la molécule organique ou complexe de coordination est choisie dans le groupe comprenant le diphénylanthracène, le rubrène, le tétracène, le cyano-méthoxy-nitro-stilbène (CMONS), le diéthyl-amino-nitro-stilbène, le naphtilimide, la rhodamine B, l'auramine O, et les complexes d'Europium.

6. Matériau selon l'une quelconque des revendications 1 à 5 dans lequel ledit au moins nano-cristal présente à sa surface au moins un type de sonde.

7. Matériau selon la revendication 6 dans lequel au moins une sonde est choisie dans le groupe comprenant les indicateurs colorés, l'ADN, les oligonucléotides ou polynucléotides, les polypeptides, les protéines, les anticorps, les sucres, les glycoprotéines et les lipides.

8. Matériau selon la revendication 6 ou 7 dans lequel au moins une sonde est greffée sur ledit au moins nano-cristal.

9. Matériau selon l'une quelconque des revendications 1 à 8 dans lequel ledit au moins nano-cristal présente un diamètre allant de 10 à 500 nm.

10. Matériau selon l'une quelconque des revendications 1 à 9 dans lequel ledit au moins un type de nano-cristal présente une distribution en taille variant au maximum de +/- 20 %.

11. Film de matériau **caractérisé en ce que** le matériau est tel que défini selon l'une quelconque des revendications 1 à 10.

12. Film selon la revendication 11 disposé sur un support solide.

13. Film selon la revendication 11 ou 12 ayant une épaisseur allant de 50 à 1500 nm.

14. Procédé de fabrication de matériau comprenant une couche inorganique et/ou organo-minérale, dans laquelle est intégré au moins un type de nano-cristal fluorescent organique ou organo-métallique émergeant de la surface de ladite couche de sorte qu'au moins une partie est en contact direct avec le milieu extérieur, ledit procédé comprenant:
a) une étape consistant à préparer au moins une couche inorganique et/ou organo-minérale comprenant ou incluant des nano-cristaux fluorescents organiques ou organo-métalliques, et
b) une étape de dissolution d'une partie de ladite couche inorganique et/ou organo-minérale obtenue à l'étape a) de manière à faire émerger au moins une partie des nano-cristaux de la couche inorganique et/ou organo-minérale ou à augmenter la surface des nano-cristaux en contact avec le milieu extérieur.

15. Procédé selon la revendication 14 dans lequel l'étape de dissolution est effectuée par des agents permettant de décaper de manière homogène la surface de la couche inorganique et/ou organo-minérale.

16. Procédé selon la revendication 15 dans lequel les agents permettant de décaper sont des solutions aqueuses basiques fortes ou faibles.

17. Procédé selon l'une quelconque des revendications 14 à 16 dans lequel l'étape de dissolution est contrôlée pour permettre une vitesse d'amincissement de la couche inorganique et/ou organo-minérale, par décapage de la surface de la couche inorganique et/ou organo-minérale, allant de 5 à 100 nm/h.

18. Procédé selon l'une quelconque des revendications 14 à 17 dans lequel la couche inorganique et/ou organo-minérale de l'étape a) est obtenue par la cristallisation de nano-cristaux organiques ou organo-métalliques fluorescents dans des couches minces de matrice sol-gel.

19. Procédé selon la revendication 18 dans lequel la matrice obtenue subit une étape de recuit.

20. Support pour biopuce, capteur et/ou senseur comprenant un substrat dont une surface comprend une couche de matériau tel que défini selon l'une quelconque des revendications 1 à 10 ou un film tel que défini selon la revendication 11, 12 ou 13.

21. Puce, capteur ou senseur comprenant au moins un matériau tel que défini selon l'une quelconque des revendications 1 à 10 ou un film tel que défini selon la revendication 11, 12 ou 13.

22. Puce, capteur ou senseur selon la revendication 21 présentant une capacité de détection d'une molécule cible pour cent molécules fluorescentes.

## Claims

1. A material comprising at least one inorganic and/or organomineral layer into which is integrated at least one type of organic or organometallic fluorescent nanocrystal that emerges from the surface of said layer so that at least one part of the nanocrystal is in direct contact with the outside environment, wherein said at least one type of nanocrystal emerges by 2 to 50% of its diameter with respect to the inorganic and/or organomineral layer.

2. Material according to claim 1 wherein the inorganic and/or organomineral layer is a sol-gel array of the Silicate, Titanate, Zirconate, Stannate, Borate, Aluminate or Yttriate type.

3. Material according to claim 1 or 2 in the form of a film.

4. Material according to any one of claims 1 to 3, wherein said at least one nanocrystal comprises at least one organic molecule or a coordination composite which fluoresces in the solid state and which is soluble in an organic solvent, with the organic molecule or coordination composite being chosen from the group comprising the fluorescent compounds of the polyaromatic series, the aromatic series, the stilbene series, the naphtilimide series, the rhodamine series, the auramine series, the perylene diimide series, the boron dipyrromethene difluoride derivatives and the rare earth complexes.

5. Material according to claim 4, wherein the organic molecule or coordination composite is selected from the group comprising diphenylanthracene, rubrene, tetracene, cyano-methoxy-nitro-stilbene (CMONS), diethyl-amino-nitro-stilbene, naphtilimide, rhodamine B, auramine O and europium complexes.

6. Material according to any one of claims 1 to 5, wherein said at least one nanocrystal has at least one type of probe on its surface.

7. Material according to claim 6, wherein at least one probe is selected from the group comprising the coloured indicators, DNA, oligonucleotides or polynucleotides, polypeptides, proteins, antibodies, carbohydrates, glycoproteins and lipids.

8. Material according to claim 6 or 7, wherein at least one probe is grafted on said at least one nanocrystal.

9. Material according to any one of claims 1 to 8, wherein said at least one nanocrystal has a diameter ranging from 10 to 500 nm.

10. Material according to any one of claims 1 to 9, wherein said at least one type of nanocrystal has a size distribution varying by +/-20% at most.

11. A film of material **characterised in that** the material is as defined according to any one of claims 1 to 10.

12. Film according to claim 11, deposited on a solid support.

13. Film according to claim 11 or 12 having a thickness ranging from 50 to 1500 nm.

14. A method for manufacturing a material comprising an inorganic and/or organomineral layer, into which is integrated at least one type of organic or organometallic fluorescent nanocrystal that emerges from the surface of said layer so that at least one part thereof is in direct contact with the outside environment, wherein said method comprises:
a) a step consisting in preparing at least one inorganic and/or organomineral layer comprising or including organic or organometallic fluorescent nanocrystals, and
b) a step of dissolving a part of said organic or organomineral layer obtained at step a) so as to make at least a part of the nanocrystals of the inorganic and/or organomineral layer emerge therefrom or to increase the surface of the nanocrystals in contact with the outside environment.

15. Method according to claim 14, wherein the dissolving step is carried out by agents allowing to homogeneously etch the surface of the inorganic and/or organomineral layer.

16. Method according to claim 15, wherein the etching agents are strong or weak basic aqueous solutions.

17. Method according to any one of claims 14 to 16, wherein the dissolving step is controlled to enable a speed ranging from 5 to 100 nm/hr for the thinning of the inorganic and/or organomineral layer, by etching the inorganic and/or organomineral layer.

18. Method according to any one of claims 14 to 17, wherein the inorganic and/or organomineral layer of step a) is obtained by crystallising organic or organometallic fluorescent nanocrystals in thin films of a sol-gel matrix.

19. Method according to claim 18, wherein the obtained matirx is submitted to a step of annealing.

20. A support for a biochip, captor and/or sensor comprising a substrate, a surface of which comprises a layer of material such as defined according to any one of claims 1 to 10, or a film such as defined according to claim 11, 12 or 13.

21. A chip, captor or sensor comprising at least one material such as defined according to any one of claims 1 to 10, or a film such as defined according to claim 11, 12 or 13.

22. A chip, captor or sensor according to claim 21 having a capacity of detecting a target molecule among one hundred fluorescent molecules.

## Patentansprüche

1. Werkstoff umfassend mindestens eine anorganische und/oder organisch-mineralische Schicht, in welche mindestens eine Art eines organischen oder organisch-metallischen fluoreszierenden Nanokristalls integriert ist, der aus der Oberfläche der Schicht herausragt, so dass mindestens ein Teil des Nanokristalls in direktem Kontakt mit dem äußeren Milieu steht, in welches mindestens eine Art eines Nanokristalls mit 2 bis 50 % seines Durchmessers in Bezug zur anorganischen und/oder organisch-mineralischen Schicht ragt.

2. Werkstoff nach Anspruch 1, in welchem die anorganische und/oder organisch-mineralische Schicht eine Sol-Gel-Matrix der Varietät Silikat, Titanat, Zirkonat, Stannat, Borat, Aluminat oder Yttriat ist.

3. Werkstoff nach Anspruch 1 oder 2, der in Form eines Films vorliegt.

4. Werkstoff nach einem beliebigen der Ansprüche 1 bis 3, in welchem mindestens der eine Nanokristall mindestens ein organisches Molekül oder einen Koordinationskomplex umfasst, der im festen Zustand fluoresziert und der in einem organischen Lösemittel löslich ist, wobei das organische Molekül oder der Koordinationskomplex aus der Gruppe ausgewählt sind, die fluoreszierende Verbindungen aus der Familie der Polyaromaten, der Familie der Aromaten, der Familie der Stilbene, der Familie der Naphtilimide, der Familie der Rhodamine, der Familie der Auramine, der Familie der Perylendiimide, Derivate von Dipyrromethenbordifluorid und Komplexe seltener Erden umfasst.

5. Werkstoff nach Anspruch 4, in welchem das organische Molekül oder der Koordinationskomplex aus der Gruppe ausgewählt werden, die Diphenylanthracen, Rubren, Tetracen, Cyanomethoxynitrostilben (CMONS), Diethylamino-nitrostilben, Naphtilimid, Rhodamin B, Auramin O und Europium-Komplexe umfasst.

6. Werkstoff nach einem beliebigen der Ansprüche 1 bis 5, in welchem mindestens der eine Nanokristall an seiner Oberfläche mindestens eine Art von Sonde aufweist.

7. Werkstoff nach Anspruch 6, in dem mindestens eine Sonde aus der Gruppe ausgewählt wird, die Farbindikatoren, DNA, Oligonukleotide oder Polynukleotide, Polypeptide, Proteine, Antikörper, Zucker, Glykoproteine und Lipide umfasst.

8. Werkstoff nach Anspruch 6 oder 7, in dem mindestens eine Sonde auf mindestens dem einen Nanokristall implantiert ist.

9. Werkstoff nach einem beliebigen der Ansprüche 1 bis 8, in dem mindestens der eine Nanokristall einen Durchmesser von 10 bis 500 nm aufweist.

10. Werkstoff nach einem beliebigen der Ansprüche 1 bis 9, in dem mindestens die eine Art Nanokristall eine Größenverteilung mit einer Variation von höchstens +/- 20 % aufweist.

11. Werkstofffilm, **dadurch gekennzeichnet, dass** der Werkstoff nach einem beliebigen der Ansprüche 1 bis 10 definiert ist.

12. Film nach Anspruch 11, der auf einen festen Träger aufgebracht ist.

13. Film nach Anspruch 11 oder 12 mit einer Stärke von 50 bis 1500 nm.

14. Verfahren zur Fertigung des Werkstoffs, der eine anorganische und/oder organisch-mineralische Schicht umfasst, in die mindestens eine Art fluoreszierendes organisches oder organisch-metallisches Nanokristall integriert ist, der aus der Oberfläche der Schicht so herausragt, dass mindestens ein Teil in direktem Kontakt mit dem äußeren Milieu steht, wobei das Verfahren folgende Schritte umfasst:
a) einen Schritt, der darin besteht, mindestens eine anorganische und/oder organisch-mineralische Schicht herzustellen, die organische oder organisch-metallische fluoreszierende Nanokristalle umfasst oder enthält, und
b) einen Schritt zur Auflösung eines Teils der in Schritt a) erhaltenen, anorganischen und/oder organisch-mineralischen Schicht, damit mindestens ein Teil der Nanokristalle aus der anorganischen und/oder organisch-mineralischen Schicht herausragt oder um die Oberfläche der mit dem äußeren Milieu in Kontakt stehenden Nanokristalle zu vergrößern.

15. Verfahren nach Anspruch 14, in dem der Auflösungsschritt durch Mittel durchgeführt wird, mit denen die Oberfläche der anorganischen und/oder organisch-mineralischen Schicht homogen gebeizt werden kann

16. Verfahren nach Anspruch 15, in dem die Mittel zum Beizen starke oder schwache basische wässrige Lösungen sind.

17. Verfahren nach einem beliebigen der Ansprüche 14 bis 16, in dem der Auflösungsschritt kontrolliert wird, um eine Verdünnungsgeschwindigkeit der anorganischen und/oder organisch-mineralischen Schicht durch Beizen der Oberfläche der anorganischen und/oder organisch-mineralischen Schicht von 5 bis 100 nm/Stunde zu ermöglichen.

18. Verfahren nach einem beliebigen der Ansprüche 14 bis 17, in dem die anorganische und/oder organisch-mineralische Schicht des Schritts a) durch Kristallisation der fluoreszierenden organischen oder organisch-metallischen Nanokristalle in den Dünnschichten der Sol-Gel-Matrix erhalten wird.

19. Verfahren nach Anspruch 18, in dem die erhaltene Matrix einem Glühschritt unterzogen wird.

20. Träger für einen Biochip, Aufnehmer und/oder Sensor umfassend ein Substrat, dessen Oberfläche eine Schicht des nach einem beliebigen der Ansprüche 1 bis 10 definierten Werkstoffs oder einen nach den Ansprüchen 11, 12 oder 13 definierten Film umfasst.

21. Chip, Aufnehmer oder Sensor, der mindestens einen nach einem beliebigen der Ansprüche 1 bis 10 definierten Werkstoff oder einen nach den Ansprüchen 11, 12 oder 13 definierten Film umfasst.

22. Chip, Aufnehmer oder Sensor nach Anspruch 21 mit der Fähigkeit, ein Zielmolekül von hundert fluoreszierenden Molekülen zu erkennen.
